# DEMANDE DE BREVET EUROPEEN

(11) **EP 4 438 020 A1**
(43) Date de publication de la demande: **02.10.2024**
(21) Numéro de dépôt: 24166579.3
(22) Date de dépôt: 26.03.2024
(51) Int. Cl.: A61F 13/49, A61F 13/494

(54) **CULOTTE MENSTRUELLE DE HAUTE PROTECTION À DOUBLURE AMOVIBLE**

(30) Priorité: 31.03.2023 FR 2303218
(71) Demandeur: Muliebris, 75018 Paris (FR)
(72) Inventeur: DELAHAYE, Amélie, 75018 Paris (FR)
(74) Mandataire: Demulsant, Xavier

(57) **Abrégé**

Culotte menstruelle (1) en tissu comprenant un devant (2), un fond (3), un dos (4), des empiècements de côté (5) et un gousset (6) situé à l'entrejambe, une doublure étant placée de manière amovible à l'entrejambe, la doublure comprenant une première face imperméable placée au contact du tissu de l'entrejambe, la doublure comprenant une face opposée perméable, la doublure présentant une partie centrale, une partie avant et une partie arrière, la partie centrale étant de largeur inférieure aux parties avant et arrière, la partie avant comprenant un bord extrême avant, la partie arrière comprenant un bord extrême arrière, la doublure étant lavable, le bord extrême avant étant arrondi, le bord extrême arrière étant en pointe.

## Description

### Domaine technique

L'invention a trait aux culottes menstruelles, destinées à être utilisées pendant la période des règles, afin d'absorber les menstruations (ou flux menstruel), ou à être utilisées en dehors de règles, notamment en cas de leucorrhées ou de légères fuites urinaires.

Par culotte on désigne ici un vêtement de dessous, dans lequel chacune des jambes s'insère séparément. Par menstruelle on désigne ici ce qui se rapporte aux menstrues, au cycle menstruel, à la menstruation. Les culottes menstruelles sont également dénommées culotte de règles.

Par menstruation on désigne ici un phénomène biologique observé chez la femme non enceinte, de la ménarche à la ménopause, caractérisé par un écoulement cyclique d'origine utérine, l'écoulement étant essentiellement composé de sang, et correspondant à l'évacuation de l'endomètre.

Les menstruations ont une durée variable, comprise entre quelques jours et deux semaines, et le flux est plus ou moins important d'une personne à l'autre.

Selon une étude de l'Inserm de 2007 (ISBN 978-2-85598-861-6), l'âge moyen des premières règles est de 12 ans en Europe, en diminution sur le dernier siècle. Le moment des premières règles, appelé ménarche, survient environ deux ans après le début de la puberté, le début de la puberté intervenant entre 8 et 13 ans. La ménopause intervient le plus souvent vers 50 ans en Europe et aux Etats-Unis (*Palacio et al*., DOI: 10.3109/13697137.2010.507886).

Les menstruations sont encore un sujet tabou, ou source de dégoût, parfois de moqueries, avec une forte demande de moyens permettant de les dissimuler. Les publicités pour les protections périodiques (ou protections menstruelles, protections intimes) promettent ainsi souvent aux femmes le confort, la discrétion, la liberté, la sécurité, la propreté, par exemple lors de la pratique d'un sport.

### Etat de la technique

Il existe sur le marché deux catégories de protections périodiques : les protections externes et les protections internes.

Les protections périodiques internes sont placées dans le vagin, et sont proposées dans diverses réalisations : tampons hygiéniques jetables à usage unique, ou lavables et réutilisables, coupes menstruelles, éponges menstruelles réutilisables.

Les protections hygiéniques externes sont portées contre la vulve, à l'intérieur d'une culotte et sont proposées dans diverses réalisations : serviettes périodiques ou protège-slips jetables à usage unique, serviettes périodiques ou protège-slips lavables et réutilisables, pétales interlabiaux, culottes menstruelles.

Les protections périodiques internes présentent un risque rare de choc toxique menstruel, pouvant entraîner de lourdes conséquences. Selon un rapport de l'Anses de décembre 2019 (saisine n°2016-SA-0108), les protections périodiques externes n'ont jamais été impliquées dans le choc toxique menstruel.

Les protections périodiques à usage unique (tampons, serviettes périodiques ou protège slip jetables) sont les protections les plus utilisées à travers le monde, et sont notamment proposées par les sociétés Procter & Gamble (marques Tampax^{®}, Always ^{®}), Johnson & Johnson (marques Vania ^{®}, Nett ^{®}, OB^{®}), Essity (marque Nana^{®}), Kimberley-Clark (marque Kotex^{®}).

Les protections périodiques à usage unique engendrent toutefois d'importants problèmes environnementaux et entraînent des dépenses importantes.

Comme indiqué dans un rapport de l'assemblée nationale de février 2020 *(rapport d'information n°2691*), le coût moyen pour les menstruations sur un mois est estimé à 10 euros, soit un coût de 4500 euros pour une vie menstruée. Ce même rapport indique qu'à l'échelle mondiale, il est estimé que 45 milliards de serviettes hygiéniques sont jetées chaque année, la dégradation de ces produits pouvant nécessiter plus de 500 ans, aucune filière de recyclage de ces produits n'existant en France.

Dans une analyse publiée en avril 2022, Blair et al. (An exploratory study of the impact and potential of menstrual hygiene management waste in the UK, Cleaner Engineering and Technology, April 2022, doi.org/10.1016/j.clet. 2022.100435*)* estiment que 28114 tonnes de protections périodiques à usage unique sont jetées chaque année au Royaume-Uni, soit dans les toilettes, soit dans les conteneurs à déchet. Lorsque jetées dans les toilettes, les protections périodiques peuvent boucher les canalisations. Lorsque jetées dans les conteneurs à déchets, les protections périodiques peuvent être incinérées avec génération de gaz à effet de serre, ou être amenées en centre d'enfouissement, la dégradation de ces produits étant très lente.

L'utilisation de protections périodiques lavables et réutilisables comme une culotte menstruelle, une serviette périodique ou un protège-slip lavable et réutilisable ou une coupe menstruelle pourrait permettre de pallier les problèmes environnementaux présentés ci-dessus.

Toutefois, les protections périodiques lavables et réutilisables actuellement commercialisées présentent plusieurs inconvénients.

Un premier inconvénient est que ce type de protection périodique nécessite un accès à un point d'eau, pour rincer ou nettoyer une coupe menstruelle par exemple, et ce dans l'intimité.

Un deuxième inconvénient est que ce type de protection périodique ne convient pas le plus souvent aux flux de sang élevés, une protection périodique interne devant notamment être utilisée en complément d'une culotte menstruelle, lorsque les règles sont abondantes.

Un troisième inconvénient est la difficulté de changer de protection périodique dans la journée, par exemple dans un lieu public ou à l'extérieur. Une personne devant changer de culotte menstruelle est obligée d'enlever ses chaussures et le bas de ses vêtements (collant, pantalon, short...), pour pouvoir enlever la culotte menstruelle et enfiler une culotte menstruelle propre.

Un quatrième inconvénient est le prix. Les culottes menstruelles sont commercialisées à des prix variant le plus souvent entre 20 et 40 euros, et il faut parfois plusieurs culottes menstruelles pour une journée de règles et plusieurs culottes menstruelles pour couvrir la totalité de la durée des règles. En France, 1.7 million de jeunes filles et femmes auraient un niveau de vie ne leur permettant pas d'accéder aux protections menstruelles nécessaires. Pour lutter contre cette précarité menstruelle, les protections périodiques réutilisables seront remboursées en France par la Sécurité sociale, à partir de 2024, pour les femmes de moins de 25 ans.

Des exemples de culottes menstruelles peuvent être trouvés dans les documents suivants : US2022387229 (Procter & Gamble, 2022), WO2022235734 (Procter & Gamble, 2023), WO2022115851 (Jockey, 2022), US2022142827 (Mast Industries, 2022), WO2021168513 (Hanes Innerwear, 2021), WO2021257900 (Thinx, 2021), WO2021155397 (Procter & Gamble, 2021), WO2021025615 (Mas Innovation, 2021).

Le document US2022096286 (Medline, 2022) décrit un sous vêtement réutilisable en coton, nylon, ou polyester. L'entrejambe du sous vêtement comprend une doublure imperméable à l'humidité, en polyéthylène, et une doublure jetable réalisée sous la forme d'un multicouche, comprenant une couche interne perméable à l'humidité, une couche absorbante, et une couche imperméable.

Le sous-vêtement décrit dans le document US2022096286 présente plusieurs inconvénients.

En premier lieu, les doublures mises en place dans l'entrejambe sont jetables, avec les frais et les impacts environnementaux qui en résultent.

En deuxième lieu, les doublures à placer dans l'entrejambe ont une forme conventionnelle, avec deux bords extrêmes arrondis ou deux bords extrêmes rectilignes, et une zone centrale plus étroite que les parties avant et arrière. Les doublures ne peuvent pas être adaptées à différents types de sous-vêtements tels que par exemple une culotte hipster, ou bikini. Par culotte hipster on désigne ici une culotte taille basse, découvrant le bas du ventre et le haut des fesses.

Un objet de l'invention est de pallier les inconvénients de l'art antérieur en proposant une culotte menstruelle, ou culotte de règles, qui soit lavable et réutilisable, la culotte menstruelle comprenant une serviette hygiénique lavable, réutilisable, et conçue pour être placée de manière amovible sur une culotte à taille basse ou à taille haute, échancrée ou montante.

Un autre objet de l'invention est de fournir une telle culotte menstruelle, dans laquelle la serviette hygiénique lavable et réutilisable est maintenue fermement à la culotte, les moyens de maintien de la serviette à la culotte ne procurant aucune gêne au porté.

Un autre objet de l'invention est de fournir une culotte répondant à l'un des objets ci-dessus, et dont le cycle de vie assure un faible impact sur l'environnement.

Un autre objet de l'invention est de fournir une culotte répondant à l'un au moins des objets ci-dessus, et pouvant être utilisée par les jeunes filles et les femmes, lors des menstruations (ou flux menstruel), ou être utilisée en dehors de règles, notamment en cas de leucorrhées ou de légères fuites urinaires, ou bien encore être utilisée par les hommes en cas de légères fuites urinaires.

A ces fins, il est proposé, selon un premier aspect, une culotte menstruelle en tissu comprenant un devant, un fond, un dos, des empiècements de côté et un gousset situé à l'entrejambe, une doublure étant placée de manière amovible à l'entrejambe, la doublure comprenant une première face imperméable placée au contact du tissu de l'entrejambe, la doublure comprenant une face opposée perméable, la doublure présentant une partie centrale, une partie avant et une partie arrière, la partie centrale étant de largeur inférieure aux parties avant et arrière, la partie avant comprenant un bord extrême avant, la partie arrière comprenant un bord extrême arrière, la doublure étant lavable, le bord extrême avant étant arrondi, le bord extrême arrière étant en pointe.

Par en pointe, on désigne ici le fait que le bord extrême arrière n'est pas rectiligne et n'est pas à rayon de courbure sensiblement constant. Par en pointe, on désigne ici notamment le fait que le bord extrême arrière a une forme générale de V. Avantageusement, le bord extrême arrière est en forme de pointe mousse, et ne comporte pas d'extrémité pouvant blesser la personne portant la culotte.

La forme de la doublure avec un bord extrême avant arrondi et un bord extrême arrière en pointe présente de nombreux avantages.

Le bord extrême avant arrondi, destiné à être placé en regard de la vulve et du bas du ventre, n'est pas blessant. En cas de déplacement accidentel de la doublure, les risques de gêne et d'irritation sont ainsi réduits.

Le bord extrême arrière en pointe, avantageusement en forme de V, remonte assez haut sur l'arrière de la culotte et assure ainsi une protection très couvrante sur l'entrejambe.

Le bord extrême arrière en pointe facilite en outre la mise en place de la doublure sur des culottes de formes différentes, à taille haute ou à taille basse, par exemples de type, bikini ou hipster.

Avantageusement, la culotte menstruelle comprend des moyens de maintien de la doublure à l'entrejambe, selon au moins deux emplacements distincts, un premier emplacement dans lequel le bord extrême avant est à une première hauteur sur le devant de la culotte, et un deuxième emplacement dans lequel le bord extrême avant est à une deuxième hauteur sur le devant de la culotte, la deuxième hauteur étant supérieure à la première hauteur.

La doublure peut ainsi être positionnée en un emplacement assurant sa présence discrète, en fonction de la forme de la culotte, notamment lorsque la culotte est à taille basse.

La doublure peut également être positionnée en fonction des activités prévues. Par exemple, la doublure peut être positionnée plus en arrière de la culotte menstruelle, pour une personne dormant sur le dos.

Selon diverses mises en oeuvre, les moyens de maintien sont choisis dans le groupe comprenant des moyens de type bande à crochets et boucles, par exemple de type Velcro^{®}, des boutons pression, ou des fentes d'insertion des bords extrêmes de la doublure au tissu de la culotte.

Avantageusement, la doublure comprend une couche absorbante réalisée en un matériau choisi parmi le coton, les viscoses cellulosiques, le bambou.

Dans certaines réalisations, la doublure comprend, pour former la première face imperméable, une feuille en matériau polymère, avantageusement un tissu polyester enduit de polyuréthane.

D'autres objets et avantages de l'invention apparaîtront à la lumière de la description de modes de réalisation, faite ci-après en référence aux dessins annexés dans lesquels :
- la figure 1 est une vue d'une culotte menstruelle selon une réalisation, la culotte étant vue sur sa partie avant, et posée à plat ;
- la figure 2 est une vue de la culotte menstruelle de la figure 1, la culotte étant vue sur sa partie arrière, et posée à plat ;
- la figure 3 est une vue de l'entrejambe d'une culotte menstruelle, selon un mode de réalisation ;
- la figure 4 est une vue d'une culotte menstruelle portée par une personne, en vue de face et en vue arrière, la largeur maximum de la doublure placée dans la culotte apparaissant sur la figure, les bords avant et arrière de trois tailles de doublure pouvant être placées dans la culotte étant rendus apparents sur la figure ;
- la figure 5 est une vue analogue à la figure 4, la culotte menstruelle étant plus échancrée que la culotte de la figure 1, les bords de la culotte de la figure 1 étant laissés apparents à fin de comparaison ;
- la figure 6 est une vue analogue à la figure 4, la culotte menstruelle étant plus montante que la culotte de la figure 1, les bords de la culotte de la figure 1 étant laissés apparents à fin de comparaison ;
- la figure 7 est une vue analogue à la figure 4, la culotte menstruelle étant de type hipster, les bords de la culotte de la figure 1 étant laissés apparents à fin de comparaison ;
- la figure 8 est une vue analogue à la figure 4, la culotte menstruelle étant de type bikini, les bords de la culotte de la figure 1 étant laissés apparents à fin de comparaison ;
- la figure 9 est une vue en plan d'une doublure pouvant être placée dans une culotte menstruelle des figures 1 à 8.

Dans la suite de cette description, le terme culotte désigne un vêtement de dessous (sous-vêtement), dans lequel chacune des jambes s'insère séparément. Le sous-vêtement est tenu à la taille, par exemple par un moyen élastique, et couvre le bas du tronc, enveloppe plus ou moins le haut de chaque cuisse ou possède deux ouvertures pour les jambes.

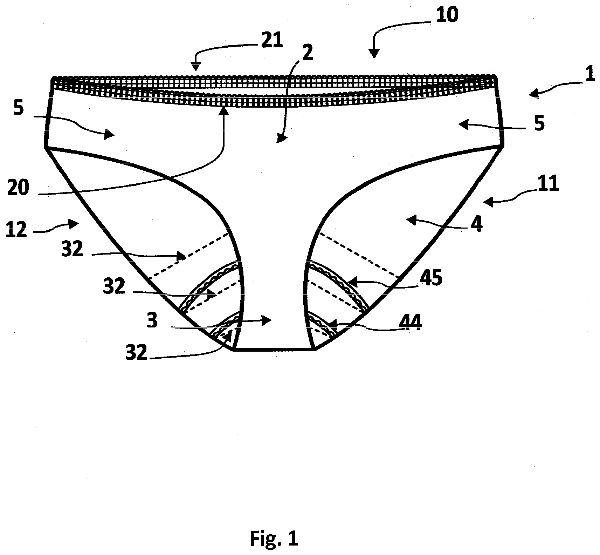

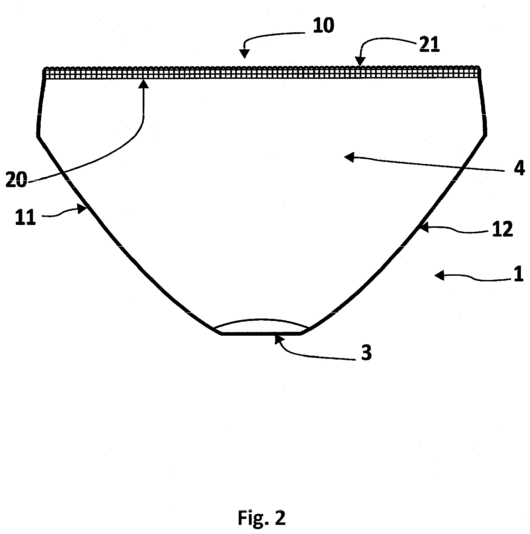

On se reporte tout d'abord aux figures 1 et 2.

La culotte menstruelle 1 représentée en figures 1 et 2 comprend un devant 2, un fond 3, un dos 4, des empiècements de côté 5 et un gousset 6 situé à l'entrejambe.

Par gousset on désigne ici une poche, avantageusement en coton, protégeant la zone intime des frottements de la culotte. Ainsi qu'il apparaitra dans la suite de cette description, le gousset 6 permet avantageusement de maintenir une doublure 13.

La forme de la culotte menstruelle 1 représentée en figures 1 et 2 est celle d'une culotte classique. Ainsi qu'il apparaitra dans la suite de cette description, les culottes menstruelles selon l'invention ne sont pas limitées aux culottes classiques, et peuvent être des culottes échancrées, à taille basse, des bikini, ou shorty.

Une doublure est rapportée, de manière amovible, à l'entrejambe.

La culotte menstruelle 1 est notamment fabriquée par assemblage d'une partie avant et d'une partie arrière, par exemple par deux coutures latérales.

Selon diverses mises en oeuvre, la culotte menstruelle 1 est formée d'au moins un corps de tissu fabriqué en coton, en nylon, en polyester, en polyamide tel que Tactel^{®}, Meryl^{®}. Le Tactel permet un touché proche de celui du coton, sans les inconvénients liés à sa fragilité et son temps de séchage.

La culotte menstruelle 1 comprend en partie supérieure une ouverture de taille 10, dont le périmètre est sensiblement égal à celui du bassin de la personne portant la culotte menstruelle 1.

La culotte menstruelle 1 comporte en partie inférieure deux ouvertures de jambes 11, 12. Les ouvertures de jambes 11, 12 sont symétriques par rapport à un plan passant par le milieu de l'ouverture de taille 10.

Le gousset 6 situé à l'entrejambe de la culotte menstruelle 1 s'étend entre une portion du bord de chaque ouverture de jambe 11,12.

Dans certaines réalisations, un élastique 20 est fixé le long de l'ouverture de taille 10, par exemple par des points de couture en zigzag.

Un ruban de dentelle 21 est avantageusement placé au bord de l'ouverture de taille 10. En variante, un ruban de tissu de couleur différente de celle du reste de la culotte menstruelle 1 est fixé au bord de l'ouverture de taille 10.

Dans certaines réalisations, un élastique est fixé le long de chacune des ouvertures de jambes 11, 12, par exemple par des points de couture en zigzag, renforçant la protection contre les fuites le long des ouvertures de jambes 11, 12, et permettant un meilleur maintien de la culotte 1 au niveau de l'ouverture des jambes 11, 12

Dans certaines mises en oeuvre, non représentées, un ruban de dentelle est avantageusement placé au bord de chaque ouverture de jambe. En variante, un ruban de tissu de couleur différente de celle du reste de la culotte menstruelle est fixé au bord de chaque ouverture de jambe.

Pour l'assemblage de la dentelle ou de rubans de tissus sur le corps de la culotte menstruelle, un fil de couture de couleur identique ou similaire au corps de tissu est avantageusement utilisé, ou bien encore un fil de couture transparent.

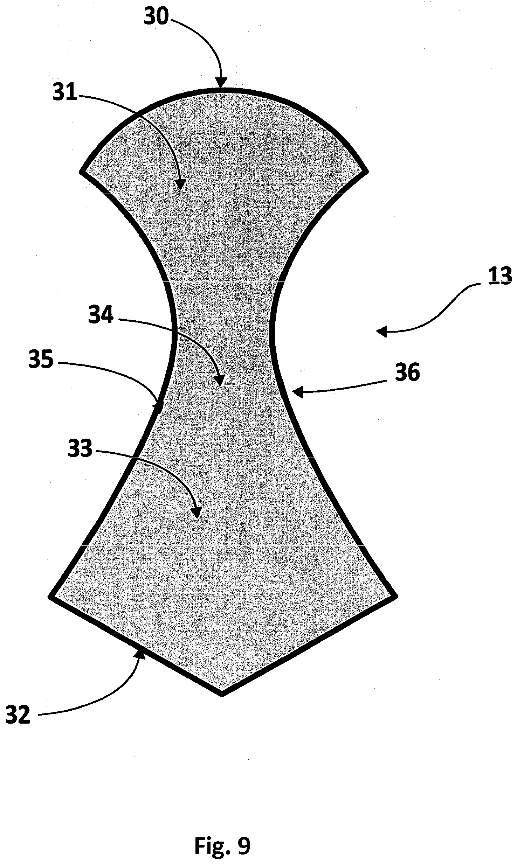

La doublure 13 (figure 9) rapportée à l'entrejambe comprend avantageusement une première face imperméable placée au contact du tissu de l'entrejambe de la culotte menstruelle 1, la doublure comprenant une face opposée perméable.

La face imperméable de la doublure 13 est avantageusement formée d'un tissu polyester revêtu de polyuréthane, par exemple par enduction. En variante, la face imperméable de la doublure est formée par un tissu technique hydrophobe à base de polyuréthane. Avantageusement, le tissu technique est choisi de manière à être respirant pour la peau du corps humain.

La face opposée perméable de la doublure 13 est avantageusement formée par une feuille de matériau en tissu drainant, par exemple en polyester, avec un poids moyen de l'ordre de 130g/m², et plus avantageusement en coton, avec un poids moyen de l'ordre de 145g/m². L'emploi de coton, avantageusement de coton biologique, permet de réduire les risques d'irritation et d'allergies.

La doublure 13 comprend une couche absorbante, s'étendant entre la face imperméable et la face opposée perméable. Selon diverses réalisations, la couche absorbante est en coton flanelle, en coton éponge, ou bien encore en micro-éponge en fibre cellulosique.

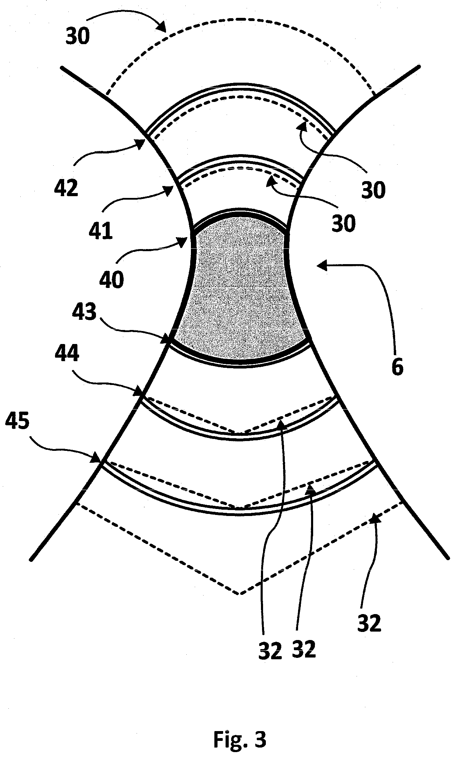

On se reporte maintenant à la figure 3 et à la figure 9.

Sur la figure 3 est représentée l'entrejambe d'une culotte menstruelle 1, les lignes en tirets faisant apparaître la position du bord extrême avant 30 de la partie avant 31 et la position du bord extrême arrière 32 de la partie arrière 33 de la doublure 13. Les termes avant et arrière sont employés ici en référence à la position de la doublure 13 à l'entrejambe de la culotte menstruelle 1. La partie avant 31 de la doublure 13 est destinée à venir à l'avant de la culotte menstruelle 1, en regard de la vulve. La partie arrière 33 de la doublure 13 est destinée à venir en regard du sillon fessier.

La doublure 13 est avantageusement lavable, en machine ou à la main, de préférence avec une eau tiède ou à froid.

Le bord extrême avant 30 de la doublure 13 est arrondi, en particulier en forme d'arc de cercle ou d'arc d'ellipse.

Le bord extrême arrière 32 de la doublure 13 est en pointe, ici en forme de V.

La doublure 13 comprend une partie centrale 34 de largeur inférieure à la largeur de la partie avant 31 et de la partie arrière 33. Dans le mode de réalisation représenté en figure 9, la doublure 13 est pourvue d'un plan de symétrie passant à mi largeur de la doublure.

La doublure 13 comporte, entre le bord extrême avant 30 et le bord extrême arrière 32, deux bords latéraux 35, 36 venant respectivement sensiblement en regard des bords de chacune des deux ouvertures de jambes 11, 12, lorsque la doublure 13 est placée à l'entrejambe.

Avantageusement, un ensemble comprenant une culotte menstruelle 1 et un ensemble de doublures 13 est livré, les doublures 13 étant identiques ou de tailles différentes.

La fourniture de doublures 13 de tailles différentes est par exemple proposée pour l'utilisation de la culotte 1 lors de menstruations à forte variation de flux, ou lorsque la doublure 13 doit être laissée en place pendant des durées variables. Par exemple, une doublure de grande taille sera mise en place durant la nuit ou lorsque la personne souhaite une protection efficace durant une longue période d'activité, ou en cas de règles abondantes.

La doublure 13 est avantageusement amovible, et n'est pas fixé à demeure par exemple par couture, sur le tissu de la culotte menstruelle 1.

La culotte menstruelle 1 comprend des moyens de maintien de la doublure 13 à l'entrejambe, selon au moins deux emplacements distincts, un premier emplacement dans lequel le bord extrême avant 30 est à une première hauteur sur le devant de la culotte 1, et un deuxième emplacement dans lequel le bord extrême avant 30 est à une deuxième hauteur sur le devant de la culotte 1, la deuxième hauteur étant supérieure à la première hauteur.

Dans le mode de réalisation représenté en figure 3, la culotte menstruelle 1 comprend des moyens de maintien d'une doublure 13 à l'entrejambe, la doublure 13 présentant trois tailles différentes.

Les moyens de maintien sont avantageusement choisis dans le groupe comprenant des moyens de type bande à crochets et boucles, notamment Velcro@, des boutons pression, ou bien encore des fentes d'insertion des bords extrêmes 30, 32 de la doublure 13 au tissu de la culotte menstruelle 1, par exemple dans des poches de type gousset.

Dans le mode de réalisation représenté en figure 3, le gousset 6 situé à l'entrejambe de la culotte menstruelle 1 comprend trois fentes 40, 41, 42 avant pour l'insertion du bord extrême avant 30 d'une doublure 13, et trois fentes 43, 44, 45 arrière pour l'insertion du bord extrême arrière 32 d'une doublure 13. Neuf tailles sont ainsi possibles pour la mise en place d'une doublure 13 à l'entrejambe de la culotte menstruelle.

Dans certaines mises en oeuvre, plus de trois doublures 13 de tailles différentes sont livrées avec la culotte menstruelle 1, offrant un nombre de possibilités élevé pour le positionnement d'une doublure à l'entrejambe, la position de la doublure étant adaptée à l'activité prévue pour la personne, et la taille de la doublure (et sa capacité d'absorption) étant adaptée à l'activité prévue de la personne ou au souhait d'une protection élevée ou au contraire modérée, en fonction des flux estimés.

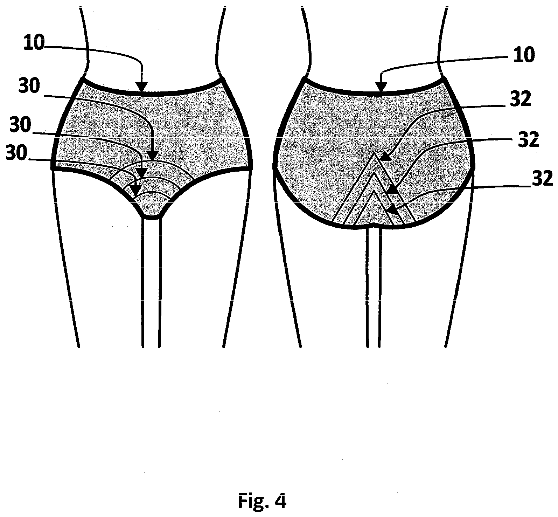

L'on se reporte maintenant aux figures 4 à 8.

En figure 4 est représentée une culotte menstruelle du type représenté en figures 1 et 2, portée par une personne, en vue de face et en vue arrière, la largeur maximum de la doublure 13 placée dans la culotte apparaissant en figure 4, les bords avant 30 et arrière 32 de la doublure 13 suivant différentes tailles pouvant être placées dans la culotte 1 étant rendus apparents sur la figure 4.

La demanderesse a constaté que les sous-vêtements féminins présentent la particularité de comporter un fond dont la forme et les dimensions sont sensiblement identiques, jusqu'à une certaine hauteur à l'avant et à l'arrière de la culotte.

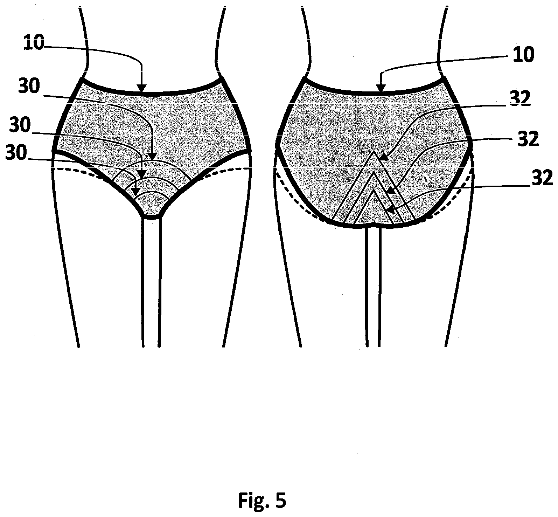

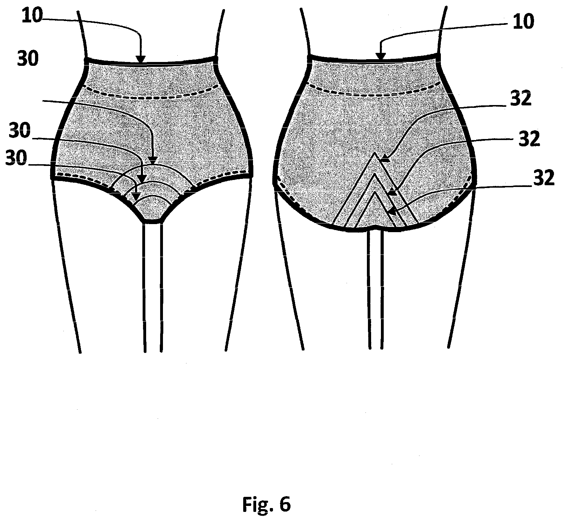

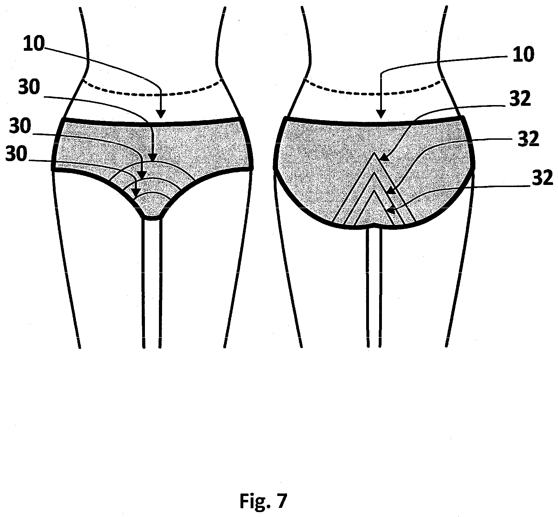

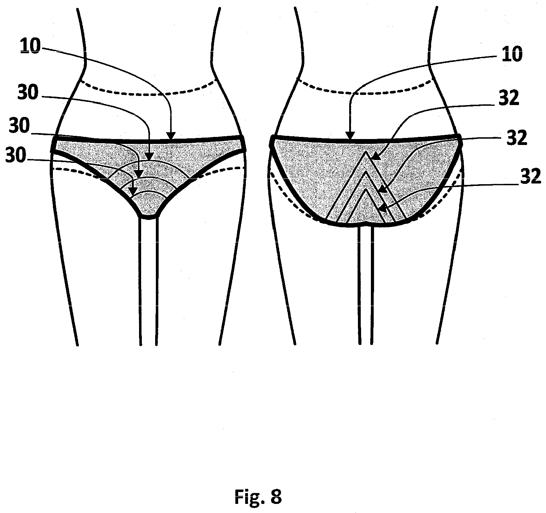

Tirant avantage de ce constat, la demanderesse propose de fixer une doublure 13 dans l'entrejambe de culottes menstruelles, cette doublure 13 étant placée avantageusement de manière amovible à l'entrejambe de culottes menstruelles 1, grâce au gousset 6, ces culottes pouvant être de formes très variées, des exemples étant présentés en figures 4 à 8 : forme classique (figure 4), culotte échancrée (figure 5), culotte montante (figure 6), hipsters (figure 7), bikini (figure 8).

La forme en pointe du bord extrême arrière 32 de la doublure 13 permet de fournir une protection pouvant remonter très haut vers l'arrière de la culotte menstruelle 1.

Les culottes menstruelles selon l'invention présentent de nombreux avantages.

Lavables, avantageusement lavables en machine, et réutilisables, les culottes menstruelles selon l'invention offrent différents positionnements de doublure à l'entrejambe, et permettent une protection adaptée aux besoins des jeunes filles et des femmes, durant leurs activités, notamment au travail ou lors de loisirs ou durant la nuit.

Le bord extrême arrière en pointe, avantageusement en forme de V, remonte assez haut sur l'arrière de la culotte et assure ainsi une protection très couvrante sur l'entrejambe. Le bord extrême arrière en pointe facilite en outre la mise en place de la doublure sur des culottes de formes différentes, à taille haute ou à taille basse, par exemple de type bikini ou hipster. Les culottes menstruelles peuvent ainsi être proposées en une large gamme, adaptée à différentes préférences et à tous les âges.

La fourniture de doublures de tailles identiques, ou de tailles différentes, avec une culotte menstruelle, permet une adaptation aux besoins, à coût réduit par rapport aux culottes menstruelles actuellement commercialisées, dans lesquelles l'entrejambe intègre un moyen absorbant, fixé à demeure. Le choix d'une taille de doublure et d'un positionnement de la doublure dans l'entrejambe permet une adaptation aux activités de la personne, et à des volumes de sang élevés.

La personne n'a pas à enlever ses chaussures et son bas (collant, pantalon, short) pour changer la doublure usagée et placer une doublure propre.

L'emploi de matériaux biosourcés (coton biologique, bambou), pour la fabrication réduit l'empreinte carbone et améliore le cycle de vie des culottes menstruelles.

## Revendications

1. Culotte menstruelle (1) en tissu comprenant un devant (2), un fond (3), un dos (4), des empiècements de côté (5) et un gousset (6) situé à l'entrejambe, une doublure (13) étant placée de manière amovible à l'entrejambe, la doublure (13) comprenant une première face imperméable placée au contact du tissu de l'entrejambe, la doublure (13) comprenant une face opposée perméable, la doublure (13) présentant une partie centrale (34), une partie avant (31) et une partie arrière (33), la partie centrale (34) étant de largeur inférieure aux parties avant (31) et arrière (33), la partie avant (31) comprenant un bord extrême avant (30), la partie arrière (33) comprenant un bord extrême arrière (32), **caractérisée en ce que** la doublure (13) est lavable, le bord extrême avant (30) étant arrondi, le bord extrême arrière (32) étant en pointe.

2. Culotte menstruelle (1) selon la revendication 1, **caractérisée en ce qu'**elle comprend des moyens de maintien de la doublure (13) à l'entrejambe, selon au moins deux emplacements distincts, un premier emplacement dans lequel le bord extrême avant (30) est à une première hauteur sur le devant de la culotte (1), et un deuxième emplacement dans lequel le bord extrême avant (30) est à une deuxième hauteur sur le devant de la culotte (1), la deuxième hauteur étant supérieure à la première hauteur.

3. Culotte menstruelle (1) selon la revendication 2, **caractérisée en ce que** les moyens de maintien sont choisis dans le groupe comprenant des moyens de type bande à crochets et boucles, des boutons pression, des fentes d'insertion des bords extrêmes de la doublure au tissu de la culotte.

4. Culotte menstruelle (1) selon l'une quelconque des revendications précédentes, **caractérisée en ce que** la doublure (13) comprend une couche absorbante réalisée en un matériau choisi parmi le coton, les viscoses cellulosiques, le bambou.

5. Culotte menstruelle (1) selon l'une quelconque des revendications précédentes, **caractérisée en ce que** la doublure (13) comprend, pour former la première face imperméable, une feuille en matériau polymère, avantageusement un tissu polyester enduit de polyuréthane.
